(19) Europäisches Patentamt

European Patent Office

Office européen des brevets

(11) Veröffentlichungsnummer: **0 087 662**
**A1**

(12) # EUROPÄISCHE PATENTANMELDUNG

(21) Anmeldenummer: 83101370.1

(22) Anmeldetag: 12.02.83

(51) Int. Cl.³: **A 61 K 9/22**
**A 61 F 1/00**
**//A61L17/00**

(30) Priorität: 25.02.82 DE 3206726

(43) Veröffentlichungstag der Anmeldung:
07.09.83 Patentblatt 83/36

(84) Benannte Vertragsstaaten:
AT BE CH DE FR GB IT LI NL SE

(71) Anmelder: Merck Patent Gesellschaft mit beschränkter Haftung
Frankfurter Strasse 250
D-6100 Darmstadt(DE)

(72) Erfinder: Kirchlechner, Richard, Dr.
Im Sennfeld 19
D-8200 Rosenheim(DE)

(72) Erfinder: Orth, Dieter, Dr.
Carl-Ulrich-Strasse 35
D-6100 Darmstadt(DE)

(72) Erfinder: Dingeldein, Elvira, Dr.
Am Spitzenpfad 9
D-6072 Dreieich(DE)

(72) Erfinder: Wahlig, Helmut, Dr.
Roemheldweg 16
D-6100 Darmstadt(DE)

(54) Pharmakadepot.

(57) Ein implantierbares und im Körper resorbierbares Pharmakadepot, das insbesondere zur lokalen Bekämpfung von Krankheiten dient, wird durch Verpressen eines schwerlöslichen Wirkstoffs mit pulverförmigem Tricalciumphosphat und gegebenenfalls einem Bindemittel erhalten.

EP 0 087 662 A1

Merck Patent Gesellschaft
mit beschränkter Haftung
Darmstadt


Pharmakadepot

Die Erfindung betrifft ein implantierbares und im Körper
resorbierbares Pharmakadepot auf Basis von Tricalciumphosphat.

Aus der deutschen Offenlegungsschrift 2807132 ist ein
Pharmakadepot auf Basis von Tricalciumphosphat bekannt,
bei dem ein Wirkstoff in die Poren einer gesinterten
Tricalciumphosphatmatrix eingelagert ist. Zur Verbesserung der protrahierten Freisetzung werden die Poren
anschließend mit einem Hilfsstoff verschlossen.

Nach der deutschen Offenlegungsschrift 28 07 132 ist es
auch bekannt, pulverförmiges Tricalciumphosphat zusammen mit einem Wirkstoff und einem Hilfsstoff zur Steuerung der Freigabekinetik zu verpressen.

Aus der deutschen Offenlegungsschrift 2843963 ist ein
Pharmakadepot auf Basis von Kollagen bekannt, bei dem
Kollagen zusammen mit einem bioresorbierbaren Bindemittel für Kollagen und einem Wirkstoff verpreßt wird,
wobei zusätzlich auch Tricalciumphosphat enthalten sein
kann.

GSPHA29 M-ad

Diese Formen haben jedoch den Nachteil, daß einmal die Herstellung kompliziert und teuer ist und daß zum anderen auch die Steuerung der Freigabe des Wirkstoffs nur schwer zu beherrschen ist.

Es bestand daher die Aufgabe, ein Pharmakadepot zu finden, das nicht nur einfach und preiswert herzustellen ist, sondern das es auch erlaubt, die Pharmakaabgabekinetik in zuverlässiger und reproduzierbarer Weise zu steuern.

Es wurde nun überraschenderweise gefunden, daß pulverförmiges Tricalciumphosphat entweder direkt oder mit Hilfe eines Bindemittels, jedoch ohne Zusatz eines die Freigabekinetik steuernden Hilfsstoffes, mit Wirkstoffen verpreßt werden kann, wobei ein Pharmakadepot entsteht, das im Körper implantiert seine Form zunächst behält und den Wirkstoff protrahiert freisetzt, auf lange Sicht jedoch im Körper resorbiert wird.

Gegenstand der Erfindung ist daher ein implantierbares und im Körper resorbierbares Pharmakadepot auf der Basis von Tricalciumphosphat, das dadurch gekennzeichnet ist, daß pulverförmiges Tricalciumphosphat mit dem Wirkstoff und gegebenenfalls einem Bindemittel verpreßt ist.

Gegenstand der Erfindung ist auch ein Verfahren zur Herstellung eines solchen Pharmakadepots.

Als Wirkstoff sind insbesondere schwer wasserlösliche Wirkstoffe bzw. Wirkstoffe in Form eines schwer wasserlöslichen Salzes geeignet. So können z. B. die besonders bevorzugten Aminoglycosidantibiotika, wie beispielsweise Gentamycin, mit Phosphorsäureestern von Flavanoiden, wie beispielsweise dem Hesperidinphosphorsäurester (auch einfach als Hesperidinphosphorsäure bezeichnet), zu schwerlöslichen Salzen umgesetzt werden. Als schwerlösliche Salze beispielsweise des Gentamycins sind weiterhin z. B. das Laurylsulfat und das Pamoat zu nennen.

Da das Antibiotikum Gentamycin bekanntlich keine einheitliche Substanz ist, sondern ein Gemisch der Verbindungen Gentamycin C1, Gentamycin C2 und Gentamycin C1a darstellt, sind auch die genannten Salze in der Regel keine einheitlichen Substanzen sondern Gemische. Da außerdem die Aminoglykosidantibiotika mehrere basische Stickstoffatome enthalten und da andererseits Hesperidinphosphorsäure (= Hesperidin-5,3'-diphosphorsäureester) und Pamoasäure (= 4,4'-Methylen-bis-(3-hydroxy-2-naphthoesäure) zweibasische Säuren sind, besteht außerdem die Möglichkeit, daß sich saure, neutrale und/oder basische Salze bilden. Alle diese möglichen Salze und ihre Gemische untereinander sind in den Definitionen "Hesperidinphosphat", "Laurylsulfat" und "Pamoat" eingeschlossen.

Bevorzugt sind die neutralen Salze und diese enthaltende Gemische. Im Falle des Gentamycinlaurylsulfats ist das Salz aus 1 mol Gentamycin und 5 mol Laurylschwefelsäure bevorzugt. Im Falle des Gentamycinhesperidinphosphat das Salz aus 2 mol Gentamycin und 5 mol Hesperidinphosphorsäure.

Die erfindungsgemäß verwendbaren schwer löslichen Salze können in an sich bekannter Weise hergestellt werden etwa durch Zusammengeben einer wässerigen Lösung eines wasserlöslichen Aminoglykosidsalzes (z. B. Gentamycinsulfat) und einer wässerigen Lösung eines wasserlöslichen Salzes der fraglichen Säure (z. B. Dinatriumsalz

GSPHA29 M-ad

des Hesperidin-5,3'-diphosphorsäureesters, Natrium-laurylsulfat, Dinatriumpamoat), zweckmäßig unter Rühren bei Raumtemperatur. Die gewünschten, in Wasser schwer löslichen Salze können nach ihrer Bildung durch Abfiltrieren, Waschen mit Wasser und Trocknen rein erhalten werden.

Der Begriff Tricalciumphosphat, der in der vorliegenden Anmeldung gebraucht wird, ist als Oberbegriff zu einer Anzahl von verschiedenen, im wesentlichen durch die chemische Formel $Ca_3(PO_4)_2$ zu beschreibenden Materialien zu verstehen, wobei das Verhältnis Calcium zu Phosphor annähernd 3 : 2 beträgt. Neben den reinen Tricalciumphosphaten wie z. B. α- oder ß-Whitlockit sollen jedoch auch die nur annähernd durch diese Formel zu beschreibenden Materialien wie z. B. Apatite oder Phosphorit umfaßt sein. Auf jeden Fall soll das Tricalciumphosphat im Körper resorbierbar sein.

Dieses Tricalciumphosphat wird in Pulverform eingesetzt, d. h. in einer Teilchengröße von etwa 0,5 bis etwa 50 µm, bevorzugt in einer Teilchengröße von etwa 0,5 bis etwa 20 µm. Auch die Wirkstoffe werden vorzugsweise in fein zerteilter, z. B. mikronisierter Form eingesetzt, falls dies möglich ist.

Zur Herstellung der erfindungsgemäßen Depots werden das Tricalciumphosphat und der Wirkstoff innig gemischt und dann verpreßt. Bei einigen der Wirkstoffe, wie z. B. dem Gentamycinlaurylsulfat, das in reiner Form eine wachsartige Konsistenz aufweist, kann auf die Zugabe eines Bindemittels völlig verzichtet werden. Trotzdem werden beim Verpressen formstabile Körper erhalten, die auch nach dem Einbringen in den Körper ihre Form behalten. Diese Zubereitungen, die auch eine sehr gute Freigabekinetik zeigen, sind daher bevorzugt.

In anderen Fällen, z. B. beim Hesperidinphosphat oder dem Pamoat oder gegebenfalls mit anderen Wirkstoffen werden zwar auch beim Verpressen stabile Formkörper erhalten, die jedoch beim Einbringen in Körperflüssigkeit leicht zerfallen. Durch Zugabe eines Bindemittels können jedoch auch hier stabile Depots erhalten werden. Geeignet sind Bindemittel, die sowohl körperverträglich als auch im Körper resorbierbar sind und dem verpreßten Depot zumindest so lange eine gewisse Stabilität im Körper verleihen, wie eine kontrollierte Freigabe von Wirkstoff aus dem Depot erwünscht ist.

Geeignete Bindemittel sind z. B. organische Bindemittel, wie z. B. Abbauprodukte des Kollagens oder Elastin. Überraschend vorteilhaft ist als Bindemittel jedoch auch Calciumsulfat zu verwenden, das wegen seiner guten Verträglichkeit, des geringen Preises wegen und wegen der sehr guten Freisetzungseigenschaften eines der bevorzugten Bindemittel darstellt.

Wenn Bindemittel eingesetzt werden, so werden in der Regel etwa 1 - 20 Gew.%, vorzugsweise etwa 3 - 10 %, insbesondere etwa 5 % verwendet. Die Verarbeitungsbedingungen, d. h. Druck und Temperatur beim Verpressen, richten sich im wesentlichen nach der Art des verwendeten Bindemittels. Falls ohne Bindemittel oder mit Calciumsulfat als Bindemittel verpreßt wird, kann in der Regel bei Raumtemperatur und Drucken von bis zu mehreren hundert bar in der Regel etwa 5 - 1200 bar, verpreßt werden, vorzugsweise bei etwa 100 - 800 bar.

Die Temperatur kann - abhängig von der Wärmestabilität des Wirkstoffes und des Bindemittels - in weiten Grenzen variiert werden. Soll das Bindemittel ein Proteinmaterial sein, z. B. ein hydrolysiertes Kollagen oder Elastin mit Molekulargewichten von etwa 2500 - 4000, dann sind Temperaturen von etwa 40 - 90 °C, vorzugsweise von 60 - 85 °C vorteilhaft.

Der Ausdruck "Wirkstoff" wird in einem sehr breiten Sinne gebraucht und umfaßt alle Artikel, die auf parenteralem Wege für die Heilung, Linderung, Behandlung und/oder Verhinderung von Gesundheitsstörungen bei Mensch und Tier vorgesehen sind oder die eine Funktion des Körpers von Mensch oder Tier zu beeinflussen vermögen.

Vor allem sind antibakterielle Wirkstoffe verschiedener Art, insbesondere Antibiotika, zu nennen. Ihr Wirkungsspektrum soll grampositive oder gramnegative Erreger oder vorzugsweise beide Gruppen umfassen. Möglichst sollen die Wirkstoffe bei den Erregern keine oder nur eine verzögerte Resistenz hervorrufen. Unter den antibakteriellen Wirkstoffen seien folgende Antibiotika beispielsweise herausgegriffen:

Aminoglykosid-Antibiotika wie Amikacin, Butirosin, Didesoxykanamycin B (DKB), Fortimycin, Gentamycin, Kanamycin, Lividomycin, Neomycin, Netilmicin, Ribostamycin, Sagamycine, Seldomycine und deren Epimere, Sisomicin, Sorbistin, Tobramycin, Streptomycine; Lincomycine wie Clindamycin, Lincomycin und Rifamycine wie Rifampicin und Rifamycin.

Die Aminoglykosid-Antibiotika, insbesondere Gentamycin, sind dabei wegen ihres breiten antibakteriellen Spektrums besonders geeignet. Insbesondere werden die schwerlöslichen Salze dieser Wirkstoffe bevorzugt.

Es ist auch möglich, zwei oder mehrere dieser Antibiotika miteinander zu kombinieren, z.B. Gentamycin mit Clindamycin; auch Kombinationen dieser Antibiotika mit anderen Wirkstoffen, z.B. mit Antiseptika, sind geeignet.

GSPHA29 M-ad

Bevorzugt sind auch andere Wirkstoffe, z.B. Antiseptika (wie Bromchlorophen, Hexetidin, Buclosamid, Salicylsäure, Cer-nitrat, Chlorhexidin, 5-Chlor-8-hydroxychinolin, Kupfer-8-hydroxy-chinolat, Acridinorange, Undecensäure, Undecoyliumchlorid, Silbersalze wie Silber-sulfadiazin, Mafenid, Nitrofurazon, Cloflucarban, Tribromsalan, Taurolin, Noxythiolin), Tuberkulostatika wie z.B. Streptomycin, Rifamycine und Isonicotinsäurehydrazid, ferner Entzündungshemmer (wie Salicylate, Phenylbutazone, Indomethacin, Ibuprofen, p-Aminophenolderivate [z.B. Acetaminophen], Pyrazolone, Hydrocortisonpalmitat) sowie Cytostatika (wie Methotrexat, Fluorouracil, Vinblastin, Doxorubicin, Prednison). Gegebenenfalls können auch Wirkstoffe verschiedener Indikationsrichtungen miteinander kombiniert werden.

Die Menge des zuzusetzenden Wirkstoffs kann in weiten Bereichen variiert werden und hängt im wesentlichen von seiner Aktivität ab. Im allgemeinen liegt die Menge des Wirkstoffes zwischen etwa 0,2 bis 20 Gew.-%, vorzugsweise bei etwa 5 - 15 Gew.-%, bezogen auf das Wirkstoffdepot.

Diese Angaben sind auf den Wirkstoff selbst bezogen. Wird ein schwer lösliches Derivat eines Wirkstoffs verwendet, z. B. die bevorzugten Laurylsulfate, Hesperidinphosphate oder Pamoate, so ist die Menge entsprechend höher, da z. B. das Gentamycinlaurylsulfat nur 26 % Gentamycinbase enthält, das Gentamycinhesperidinphosphat gar nur 13 % Gentamycinbase.

Die Freisetzung des Wirkstoffs aus dem Depot kann in an sich bekannter Weise entweder in vitro, z. B. durch Elution mit Wasser oder wässerigen Pufferlösungen oder tierischem oder menschlichem Serum, bestimmt werden oder in vivo nach Implantation durch Messen der Ausscheidung im Urin oder durch Messen der zeitlichen Veränderung der Konzentration im Serum oder in Geweben.

Es hat sich gezeigt, daß insbesondere schwer lösliche Wirkstoffe bzw. schwer lösliche Derivate von Wirkstoffen wie z. B. das Gentamycinlaurylsulfat, das Hesperidinphosphat und das Pamoat eine sehr vorteilhafte Freisetzungskinetik besitzen. Die Freisetzung steigt dabei zunächst von relativ geringen Werten zu einer über einen langen Zeitraum gleich bleibenden Freisetzung von gut wirksamen Konzentrationen an, um dann nach Erschöpfung des Depots wieder abzufallen. Falls es erwünscht ist, auch in der Startphase eine hohe oder gar höhere Wirkstoffkonzentration in der Umgebung des Depots zu erzeugen, hat es sich als vorteilhaft erwiesen, neben dem schwer löslichen Wirkstoff noch eine ausreichende Menge des Wirkstoffes in leicht löslicher Form beizumischen.

Z. B. kann durch ein Depot, das eine Mischung von etwa 10 Gew.% (bezogen auf die freie Base) Gentamycin Laurylsulfat und etwa 2 - 5 % Gentamycinsulfat enthält, eine Freisetzung erreicht werden, bei der anfänglich eine relativ hohe Wirkstoffkonzentration erzielt werden, die dann zu einer gleichbleibend hohen und gut wirksamen Konzentration abfällt.

Mit dem Ausdruck "schwer löslicher Wirkstoff" ist in der Regel ein Wirkstoff zu verstehen, der eine Löslichkeit von etwa 1 - 1000 mg/l besitzt. Diese absolute Löslichkeit gibt jedoch nur einen groben Anhaltspunkt und kann im Einzelfall sowohl über- als auch unterschritten werden. Entscheidend ist, daß die Löslichkeit in einem bestimmten Verhältnis zur wirksamen Menge des betreffenden Wirkstoffes steht. Bei Wirkstoffen, die in äußerst geringen Mengen wirksam sind, bedeutet "schwerlöslich" im Sinne dieser Erfindung eine sehr viel niedrigere absolute Löslichkeit als bei Wirkstoffen, die erst in größeren Konzentrationen wirksam werden. In der Regel sollte ein schwerlöslicher Wirkstoff im Sinne dieser Erfindung eine absolute Löslichkeit besitzen, die etwa das 100- bis 500-fache der wirksamen Konzentration beträgt. Bei Antibiotika, die MHK-Werte (MHK = minimale Hemm-Konzentration) in der Größenordnung von etwa 1 µg/ml besitzen, bedeutet demnach "schwerlöslich" eine Löslichkeit von etwa 100 - 500 mg/l. Bei in geringeren Mengen wirksamen Verbindungen genügt jedoch schon eine Löslichkeit von wenigen Milligramm pro Liter. Da das erfindungsgemäße Pharmakadepot vorzugsweise an dem Ort eingesetzt wird, an dem es wirksam werden soll, und somit keine Verdünnung des Wirkstoff durch Transport im Körper auftritt, können auch Wirkstoffe mit gutem Erfolg eingesetzt werden, die in der Literatur als praktisch unlöslich beschrieben sind.

Das erfindungsgemäße Pharmakadepot kann in den Körper eingebracht und dort belassen werden, da alle Bestandteile im Körper resorbierbar bzw. abbaubar sind. Vorteilhafterweise braucht das Depot im allgemeinen nur ein einziges Mal appliziert zu werden. Es ist dabei möglich, das Depot dort zu applizieren, wo der Wirkstoff eingreifen soll. Dies ist insbesondere bei der Bekämpfung von lokalen Infekten von Vorteil, da nicht der gesamte Körper z. B. mit Antibiotika überschwemmt wird, sondern nur an den tatsächlich infizierten oder infektionsgefährdeten Stellen eine wirksame Konzentration erzeugt wird.

GSPHA29 M-ad

Antibiotikahaltige Wirkstoffdepots nach der Erfindung können daher mit Vorteil z. B. zur Infektionsprophylaxe bei verschmutzten ausgedehnteren Weichteilwunden oder bei Trümmerzonen offener Knochenbrüche, wie sie beispielsweise bei Unfällen häufig auftreten, eingesetzt werden oder zum Ausfüllen von infizierten Wundhöhlen. Bei der Behandlung von infizierten Knochenhöhlen wird durch das Tricalciumphosphat auch die Knochenneubildung günstig beeinflußt.

Das erfindungsgemäße Pharmakadepot kann dabei in beliebiger, dem Verwendungszweck angepaßter Form, eingesetzt werden z. B. in Form von Tabletten, Kugeln, Zylindern, Stäben, Platten oder auch als Granulat. Durch die einfache und preiswerte Herstellung und die günstigen Freisetzungseigenschaften steht damit ein vorteilhaftes neues Pharmakadepot zur Verfügung.

Beispiele:

1.    Herstellung von schwerlöslichen Wirkstoffen

Beispiel 1.1

Eine Lösung von 7,07 g (10 mmol) Gentamycinsulfat in 200 ml Wasser wird bei 20 °C unter Rühren mit einer Lösung von 20,4 g (25 mmol) Hesperidin-5,3'-Diphosphorsäureester-Dinatriumsalz in 600 ml Wasser versetzt. Man rührt noch eine Stunde, saugt das erhaltene Gentamycinhesperidinphosphat ab, wäscht mit Wasser nach und trocknet über KOH. Schmelzpunkt 227 - 229° C (Zersetzung); IR-Spektrum (in KBr): 3410, 2950, 1637, 1572, 1510, 1440 cm$^{-1}$.

Beispiel 1.2

Eine Lösung von 7,07 g Gentamycinsulfat in 200 ml Wasser wird bei 20 °C unter Rühren mit einer Lösung von 10,8 g (25 mmol) Dinatrium-Pamoat in 800 ml Wasser versetzt. Man arbeitet analog Beispiel 1.1 auf und erhält Gentamycinpamoat, Schmelzpunkt 228 - 230 °C (Zersetzung); IR-Spektrum (in KBr): 3430, 3070, 2950, 1635, 1555, 1505, 1450 cm$^{-1}$.

Beispiel 1.3

Eine Lösung von 7,07 g Gentamycinsulfat in 200 ml Wasser wird bei 20 °C unter Rühren mit einer Lösung von 14,4 g (50 mmol) Natrium-Laurylsulfat in 500 ml Wasser versetzt. Man arbeitet analog Beispiel 1.1 auf und erhält Gentamycinlaurylsulfat, Schmelzpunkt 190 - 193 °C; IR-Spektrum (in KBr): 3440, 2940, 2865, 1615, 1515, 1462 cm$^{-1}$.

Beispiel 1.4

Eine 500 mg (0,813 mmol) Neomycinbase entsprechende Menge Neomycinsulfat wird in 2 ml Wasser gelöst und unter Rühren zu einer Lösung von 1.99 g (2.44 mmol) Hesperidinphosphorsäurenatriumsalz in 10 ml Wasser gegeben, wobei ein schwerlösliches Salz ausfällt. Man saugt ab, wäscht mit Wasser nach und trocknet i. Vak.; Schmp. 228 - 230° C (Zers.).

Beispiel 1.5

Die 1,0 g (1,624 mmol) Paromomycinbase entsprechende Menge Paromomycinsulfat in 5 ml Wasser und 3,3 g (4,06 mmol) Hesperidinphosphorsäurenatriumsalz in 10 ml Wasser gelöst, werden unter Rühren vereinigt.

Das schwerlösliche Salz wird abgesaugt, mit Wasser gewaschen und i. Vak. getrocknet; Schmp. 219 - 222°C (Zers.).

Beispiel 1.6

Eine 0,1 g (o,223 mmol) Sisomycinbase entsprechende Menge Sisomycinsulfat in Wasser gelöst (5 Ampullen zu 2 ml) und 0,455 g (0,558 mmol) Hesperidinphosphorsäurenatriumsalz in 5 ml Wasser, werden unter Rühren vereinigt. Nach Absaugen des ausgefallenen Salzes, Waschen mit Wasser und Trocknen, Schmp. 220 - 221° C (Zers.).

Beispiel 1.7

Die 0,5 g (0,854 mmol) Amikacinbase entsprechende Menge Amikacinhydrogensulfat in Wasser (5 Ampullen zu 2 ml) wird unter Rühren mit einer Lösung von 1,39 g (1,7 mmol) Hesperidinphosphorsäurenatriumsalz in 10 ml Wasser vereinigt. Das ausgefallene Salz wird dann abgesaugt, mit Wasser gewaschen und i. Vak. getrocknet; Schmp. 226 - 229° C (Zers.).

Beispiel 1.8

Die 0,1 g (0,214 mmol) Tobramycinbase entsprechende Menge Tobramycinsulfat in 5 ml Wasser und 0,435 g (0,534 mmol) Hesperidinphosphorsäurenatriumsalz in 5 ml Wasser werden unter Rühren vereinigt. Nach Absaugen des ausgefallenen Produktes und Waschen mit Wasser wird i. Vak. getrocknet; Schmp. 228° C (Zers.).

Beispiel 1.9.

Eine 0,25 g (0,554 mmol) Dibekacinbase entsprechende
Menge Dibekacinsulfat in 5 ml Wasser (5 Ampullen zu
1 ml) und 1,13 g (1,384 mmol) Hesperidinphosphorsäurenatriumsalz in 5 ml Wasser werden unter Rühren
vereinigt. Das ausgefallene Salz wird abgesaugt,
mit Wasser gewaschen und i. Vak. getrocknet;
Schmp. 230° C (Zers.).

Beispiel 1.10

Man löst 2,44 g (3 mmol) Hesperidinphosphorsäureesternatriumsalz in 20 ml Wasser und versetzt unter
Rühren mit einer Lösung von 1,457 g (1 mmol)
2 Streptomycin · 3 Schwefelsäure. Das ausgefallene
Salz wird abgesaugt, mit Wasser gewaschen und i.
Vak. getrocknet, Schmp. 212 - 213$^d$ C (Zers.).

Beispiel 1.11

578 mg (1 mmol) Chlorhexidinhydrochlorid in 100 ml
Methanol + 100 ml Wasser gelöst, wird mit 50 ml einer
Lösung von 814 mg (1 mmol) Hesperidinphosphorsäureesternatriumsalz in Wasser versetzt, das ausgefallene
Salz abgesaugt, mit Wasser gewaschen und i. Vak.
getrocknet; Schmp. 210 - 212° C (Zers.).

2.   Herstellung von Pharmakadepots

Beispiel 2.1

3,0 g Gentamycinlaurylsulfat und 3,0 g penta-
Calciumhydroxidtriphosphat werden innig gemischt

GSPHA29 M-ad

- 14 -                                    0087662

und mit einer Tablettenpresse bei Raumtemperatur
und einem Preßdruck von etwa 100 bar zu Tabletten
gepreßt.

Beispiel 2.2

3,0 g Gentamycinhesperidinphosphat, 3,0 g penta-
Calciumhydroxidtriphosphat und 300 mg Calciumsulfat
werden innig gemischt und in einer Tablettenpresse
bei Raumtemperatur und einem Preßdruck von etwa
100 bar zu Tabletten gepreßt.

Beispiel 2.3

3,0 g Gentamycinpamoat, 3,0 g penta-Calciumhydroxidtriphosphat und 1 g Proteinpulver werden innig gemischt und mit einer Tablettenpresse unter Erwärmung
auf etwa 80 $^{\circ}$C bei einem Preßdruck von etwa 100 bar
zu Tabletten gepreßt.

Beispiel 2.4

5,0 g Gentamycinhesperidinphosphat (19 % Gehalt an
Gentamycinbase), 5,0 g penta-Calciumhydroxidtriphosphat, 500 mg Calciumsulfat und 78,2 mg Gentamycinsulfat (entsprechend 47,5 mg Gentamycinbase) werden
innig gemischt, gesiebt und bei 100 bar zu Tabletten
verpreßt.

Das Depot enthält 5 % freies Gentamycin, bezogen auf
den Gesamtgentamycingehalt. Depots mit 10 bzw. 20 %
freiem Gentamycin werden analog mit entsprechend
größeren Mengen Gentamycinsulfat hergestellt.

GSPHA29 M-ad

Beispiel 2.5

50 g Indomethacin, 50 g Calciumhydroxidtriphosphat und 5 g Calciumsulfat werden innig gemischt und bei Raumtemperatur und einem Druck von etwa 100 bar zu Tabletten gepreßt. Gegenüber freiem Indomethacin wird eine deutlich retardierte Freisetzung beobachtet.

Beispiel 2.6

50 g Methotrexat, 50 g Calciumhydroxidtriphosphat und 5 g Calciumsulfat werden innig gemischt und bei Raumtemperatur und einem Druck von etwa 100 bar zu Tabletten gepreßt.

Beispiel 2.7

5 g Neomycin B-Hesperidinphosphat, 5 g Pentacalciumhydroxidtriphosphat und 500 mg Calciumsulfat werden innig gemischt und bei einem Druck von etwa 100 bar bei Raumtemperatur zu Tabletten gepreßt.

Beispiel 2.8

5 g Paromomycin-Hesperidinphosphat, 5 g Pentacalciumhydroxidtriphosphat und 500 mg Calciumsulfat werden innig gemischt und bei einem Druck von etwa 100 bar bei Raumtemperatur zu Tabletten gepreßt.

Beispiel 2.9

5 g Sisomycin-Hesperidinphosphat, 5 g Pentacalciumhydroxidtriphosphat und 500 mg Calciumsulfat werden innig gemischt und bei einem Druck von etwa 100 bar bei Raumtemperatur zu Tabletten gepreßt.

Beispiel 2.10

5 g Amikacin-Hesperidinphosphat, 5 g Pentacalciumhydroxidtriphosphat und 500 mg Calciumsulfat werden
innig gemischt und bei einem Druck von etwa 100 bar
bei Raumtemperatur zu Tabletten gepreßt.

Beispiel 2.11

5 g Tobramycin-Hesperidinphosphat, 5 g Pentacalciumhydroxidtriphosphat und 500 mg Calciumsulfat werden
innig gemischt und bei einem Druck von etwa 100 bar
bei Raumtemperatur zu Tabletten gepreßt.

Beispiel 2.12

5 g Dibekacin-Hesperidinphosphat, 5 g Pentacalciumhydroxidtriphosphat und 500 mg Calciumsulfat werden
innig gemischt und bei einem Druck von etwa 100 bar
bei Raumtemperatur zu Tabletten gepreßt.

Beispiel 2.13

5 g Streptomycin-Hesperidinphosphat, 5 g Pentacalciumhydroxidtriphosphat und 500 mg Calciumsulfat
werden innig gemischt und bei einem Druck von etwa
100 bar bei Raumtemperatur zu Tabletten gepreßt.

Beispiel 2.14

5 g Chlorhexidin-Hesperidinphosphat, 5 g Pentacalciumhydroxidtriphosphat und 500 mg Calciumsulfat
werden innig gemischt und bei einem Druck von etwa
100 bar bei Raumtemperatur zu Tabletten gepreßt.

GSPHA29 M-ad

Merck Patent Gesellschaft
mit beschränkter Haftung
Darmstadt

Patentansprüche

1.  Implantierbares und im Körper resorbierbares
Pharmakadepot auf der Basis von Tricalciumphosphat,
dadurch gekennzeichnet, daß pulverförmiges Tricalciumphosphat mit dem Wirkstoff und gegebenenfalls einem Bindemittel verpreßt ist.

2.  Pharmakodepot nach Anspruch 1, dadurch gekennzeichnet, daß ein schwerlöslicher Wirkstoff enthalten ist.

3.  Pharmakadepot nach Anspruch 1 oder 2, dadurch gekennzeichnet, daß als Wirkstoff ein Aminoglykosidantibiotikum in Form eines schwer wasserlöslichen Salzes
enthalten ist.

4.  Pharmakadepot nach Anspruch 3, dadurch gekennzeichnet, daß Gentamycin in Form eines schwer löslichen
Salzes enthalten ist.

5.  Verfahren zur Herstellung eines implantierbaren und
im Körper resorbierbaren Pharmakadepots auf der
Basis von Tricalciumphosphat, dadurch gekennzeichnet, daß pulverförmiges Tricalciumphosphat mit dem Wirkstoff und gegebenenfalls einem Bindemittel verpreßt wird.

6. Verfahren nach Anspruch 5, dadurch gekennzeichnet, daß Tricalciumphosphat mit einer Teilchengröße von 0,5 bis 50 /um mit 0,2 - 20 Gew.% des Wirkstoffs und gegebenenfalls 1 - 20 Gew.% eines Bindemittels vermischt wird und bei einer Temperatur von Raumtemperatur bis etwa 200 $^{o}$C und einem Druck von etwa 5 - 1200 bar verpreßt wird.

GSPHA29 M-ad

**Europäisches Patentamt**

# EUROPÄISCHER RECHERCHENBERICHT

**0087662**
Nummer der Anmeldung

EP 83 10 1370

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,Y | EP-A-0 003 979 (BATTELLE) <br> * Ansprüche 1,8; Seite 3, Zeilen 6-26 * | 1,5,6 | A 61 K 9/22 <br> A 61 F 1/00 // <br> A 61 L 17/00 |
| | --- | | |
| Y | EP-A-0 016 906 (MERCK) <br> * Seite 6, Zeilen 5-26; Seite 18, Zeilen 10-18; Seite 20, Beispiel 4 * | 1,5,6 | |
| | --- | | |
| D,A | FR-A-2 438 479 (MERCK) | | |
| | --- | | |
| P | EP-A-0 058 867 (MUNDIPHARMA) <br> * Ansprüche 1,9 * | 1 | |
| | --- | | |
| P | EP-A-0 061 108 (MUNDIPHARMA) <br> * Ansprüche 1,13 * | 1 | RECHERCHIERTE SACHGEBIETE (Int. Cl. 3) |
| | ----- | | A 61 K 9/22 <br> A 61 F 1/00 <br> A 61 L 17/00 |

Der vorliegende Recherchenbericht wurde für alle Patentansprüche erstellt.

| Recherchenort <br> DEN HAAG | Abschlußdatum der Recherche <br> 08-06-1983 | Prüfer <br> PELTRE CHR. |
|---|---|---|